# EUROPEAN PATENT SPECIFICATION

(11) **EP 1 931 262 B1**
(45) Date of publication and mention of the grant of the patent: **16.11.2016**
(21) Application number: 06803755.5
(22) Date of filing: 18.09.2006
(51) Int. Cl.: A61B 90/00, A61B 5/05, A61B 6/00, G01J 3/28, G01J 3/02, G01N 21/27, G01N 21/31

(54) **DISPOSABLE CALIBRATION-FIDUCIAL MARK FOR HYPERSPECTRAL IMAGING**
EINWEG-KALIBRATIONSBILDKOORDINATEN-MARKIERUNG FÜR HYPERSPEKTRALE DARSTELLUNG
REPERE D'ETALONNAGE A USAGE UNIQUE POUR IMAGERIE HYPERSPECTRALE

(30) Priority: 16.09.2005 US 717188 P
(43) Date of publication of application: 18.06.2008
(73) Proprietor: HyperMed Imaging, Inc., Memphis TN 38125 (US)
(72) Inventor: PANASYUK, Svetlana, V., Lexington, MA 02420 (US); FREEMAN, Jenny, E.,, Weston, MA 02493 (US); HOPMEIER, Michael, J., Mary Esther, FL 32569 (US); OYLER, Creighton, J., San Francisco, CA 94129 (US)
(74) Representative: Grund, Martin
(86) International application number: PCT/US2006/036223
(87) International publication number: WO 2007/035597

(56) References cited:
- US-A- 4 857 716
- US-A- 5 016 173
- US-A- 5 016 173
- US-A- 5 924 981
- US-A1- 2002 022 273
- US-A1- 2005 007 584
- US-A1- 2005 007 584
- US-B1- 6 937 885
- US-B1- 6 937 885

## Description

### Background

### 1. Field of the Invention

This invention is directed to spectral-imaging calibration and registration for use with medical patients. In particular, the invention is directed to systems for calibrating hyperspectral/multispectral imaging of patients.

### 2. Description of the Background

A typical color picture (photographic image) usually provides more information than a black-white image enabling the viewer to better distinguish among objects. This is a simple example of a spectroscopic image where the ability of objects to reflect light at three wavelengths ranges (red, green, and blue) is depicted. Based on the intensity of such reflections, objects such as green and red apples can be distinguished and evaluated according to chemical composition based on color.

Spectroscopy, like many other analytical imaging techniques, has undergone an evolution in terms of the types of research fields in which it is being utilized. From its early beginnings, it was, and continues to be, a plentiful research field in the hands of physicists. Later, chemists discovered that spectroscopy was a useful tool for the investigation of complex molecular structures. Later still, biologists discovered the usefulness of spectroscopy in the analysis of the structures of biomolecules.

Spectroscopy has emerged into medicine. The natural progression of spectroscopy into medicine has paralleled another spectroscopic technique, MRI. The original investigations into problems of medical significance were based on the premise that the biochemistry of a tissue must change before changes in anatomy or morphology, the current standard criteria for many diagnoses, become apparent, and that these biochemical changes are contained within the spectral signature. Therefore, chemical changes correlated with change sin biology, metabolism or physiology (some associated with disease states) are apparent by spectroscopic analyses prior to any clinical appearance.

Spectra are known to be sensitive to subtle changes in molecular composition and conformation. Spectroscopic analysis of biomolecules is a well established field; and, as a chemist knows, the spectrum of a molecule forms a unique "fingerprint" of that compound. This maxim generally holds true for pure compounds. Tissues, be they human or animal, are a complex and highly variable mixture of compounds. The typical spectra obtained from tissue are a weighted average of the spectral features of each of the chemical constituents being sampled within a given sample volume, and as such, these spectra contain information about the chemical state of the entire sample.

A major obstacle in medical spectroscopy has been sorting out useful diagnostic information from the inter and intra-sample variability. It is typically insufficient to take a spectrum from a healthy piece of tissue and a diseased piece of tissue, compare them, and make valid claims regarding their disease state. One should take into account the range of data and biological states which occur over a population, as well as the intrinsic variability of tissue spectra during such analysis. This process requires either large, statistically relevant numbers of spectra or a methodology that takes into account the intrinsic inter-sample variability and spatial heterogeneity.

Spectroscopic investigations of medical interest can be roughly divided into three major areas: clinical chemistry, where the goal is to provide a quantitative analysis of blood, other body fluid, tissue samples, in vitro samples, ex-vivo samples, body surfaces or other conditions or substances; pathology, which attempts to provide an alternative pathological assessment of a tissue biopsy; and *in vivo* analyses, where the analysis is done without the need for an invasive procedure. The vision of having a small, inexpensive, portable instrument capable of making a rapid, non-invasive assessment of some relevant medical parameter has provided the driving force behind the application of visible and near-IR spectroscopic techniques to issues of medical interest.

The optical properties of tissue are governed by a number of factors, including the bulk scattering properties as well as their absorbance. Variations in tissue or blood analyte composition and/or concentrations will affect visible and near-IR tissue absorbance, while changes in the tissue blood-volume will affect the scattering properties. The interpretation of *in-vivo* reflectance data is further complicated in that most physical situations which modify tissue absorbance also affect tissue scattering. Visible and near-IR spectroscopic methods have been used for decades in operating theatres in the form of pulse oximeters. These simple systems utilize the different oxy-and deoxy-hemoglobin absorption bands to determine arterial oxygen saturation.

Unlike single point spectroscopy, hyperspectral imaging (HSI) allows mapping of regional variations in hemodynamic parameters in response to tissue perfusion. Unlike infrared thermography, HSI does not map the thermal emission of the tissues. Instead, it relies on a spectroscopic image that, through interpretation provides representations of hemoglobin oxygen saturation of that tissue. One application of HSI is in the determination of tissue viability following plastic surgery. Tissue which has insufficient oxygenation to remain viable is readily apparent from oxygen saturation maps calculated from near-IR spectral images acquired immediately following surgery; clinical signs of the loss of viability do not become apparent for 6 to 12 hours post-surgery.

HSI, in one guise or another, has become a useful tool for the investigation of spatial heterogeneity in spectral properties in a variety of fields of study ranging from astronomy to medicine. Used for decades in airplane and satellite mounted systems for the mapping of land use and soil types, it has moved in the last five years into a large number of application areas. Of particular interest here is the use of HSI in the fields of biophysics and medicine. The combination of spectroscopic imaging and microscopy has proved very useful in the investigation of the spectral properties of slices of tissue. In addition to being useful for the investigation of microscopic structures, HSI systems for imaging macroscopic structures have been shown to be useful in the monitoring of the spatial distribution of skin oxygenation. HSI, however, allows mapping of the regional variations in hemodynamic parameters in response to tissue perfusion.

When evaluating a chemical composition with spectral imaging using many light wavelengths, images must be collected or constructed at each wavelength. Over the years, various methods have been devised to collect spectroscopic images. Generally in such cases, a spectroscopic imaging experiment generates data in the form of what is often called a spectral image cube, spectroscopic imaging data cube or just hypercube. This is a three dimensional array of data, consisting of two spatial dimensions (the imaging component), and one spectral dimension. The array can be thought of as an array of spatially resolved individual spectra, with every pixel in the image consisting of an entire spectrum, or as a series of spectrally resolved images. In either representation, the 3D data cube can be treated as a single entity and can contain spatial and spectral information about the sample from which it was acquired.

A hyperspectral, multispectral or selected spectral imaging system can acquire images at hundreds of wavelengths, many wavelengths or at specially selected wavelengths or combinations of wavelengths as opposed to the three colors seen by humans. The added sensitivity from multiple wavelengths allows such devices to accurately measure properties of the sample, such as hydration or oxygenation. However, the devices are also sensitive to many sources of variance, including environmental conditions (such as lighting, temperature, humidity), shading and shadowing due to object shape, and object or camera motion.

Typically, the instrument is aimed at a calibration target having known optical properties. Light is then scattered or reflected by the calibration target and received back in the instrument. Because the calibration target has known optical properties, the instrument is able to perform a calibration operation to ensure that the instrument continues to deliver accurate results.

The structure, chemical composition and oxidative state of living tissue may be determined by illuminating volume elements, or voxels, of the tissue with electromagnetic radiation (such as visible light) of one or several wavelengths, collecting the light reflected, transmitted, scattered and emitted by each voxel at one or several wavelengths and then using various mathematical means to analyze the multispectral data collected from individual and multiple voxels. For example, tissue in the human earlobe is transilluminated with light of a variety of wavelengths. The transmitted light is then measured and relative proportions of the two pigments carried in red blood cells, hemoglobin and deoxyhemoglobin, are calculated. These calculations form the basis for calculating the fraction of the maximum oxygen carrying capacity of the hemoglobin molecule, called the oxygen saturation or O₂Sat.

The documents US 2005/007584 A1 and US 5,016,173 A disclose a disposable imaging reference for hyperspectral imaging of a tissue surface having the characteristics of the preamble of appended claim 1.

### Summary

Embodiments alleviate or overcome the problems and disadvantages associated with current strategies and designs and provides new tools and methods for acquiring spectral images with improved quality. The present invention also can provide previously unavailable information about tissue signatures to assist in diagnosis and therapy. Embodiments provide various apparatuses for obtaining tissue signatures, particularly that have a first surface for contacting the tissue and at least a second surface having an characteristic pattern of sharp contrast color peaks and wherein two or more of the peaks have different identifying constant color standards at one or more wavelength bands. The term "sharp contrast" is known to the skilled artisan and generally means at least two different colors that are easily distinguished (i.e. without ambiguity) by the equipment such as that described in U.S. No. 7,013,172 ("Hyperspectral imaging calibration device"). Embodiments also provide methods for obtaining such spectral images with improved quality.

The invention is defined in the characterizing portion of appended claim 1. The invention is directed to a disposable fiducial mark for use with hyperspectral imaging comprising a spatial pattern with sharp peaks; a set of reflectance standards at one or more wavelength bands; a means for attaching to tissue; and a means for removal. A means for attaching and removing may be an adhesive present on a surface of the fiducial mark, an adhesive applied to the surface before application, a mechanical or biological material on the surface that bonds with organic material of the tissue, such as a blood clotting factor, skin component, or material that chemically reacts with a fluid from or protein of the surface, a clamp, a bandage, a pin, or Velcro. In an embodiment the adhesive means is a material known in the field of treatment of burn patients that desirably is applied to a severe burn. In this embodiment, the fiduciary mark may comprise be a bandage or other therapeutic material (such as an ex vivo tissue culture or preparation) that both treats as well as serves as a fiduciary marker. The mark may be sterile, have directional asymmetry, and/or include an area of low reflectivity. Desirably, the mark has a unique spectral signature to allow identification, particularly in a large hospital environment, wherein the mark may serve as a primary or secondary backup of patient identification. In an embodiment, the fiduciary mark has a standard predetermined code of colors, color/light intensities and or shapes that can be decoded to reveal alphanumeric information such as patient name and other bioparameters. A bar code may be included.

Also disclosed herein is a method for aligning hyperspectral images comprising aligning images with the mark and acquiring images from at least one wavelength. The method may also adjust the images with software and a computer algorithm. Most desirably, image information obtained by a camera is used to determine the physical orientation of the mark (and/or of a patient body) with respect to the camera. For example, in MRI, and other imaging systems, an additional use of the fiduciary mark can allow real time monitoring of patient/body part placement. In an embodiment, an algorithm in a computer accepts image data looks for a pattern of the data and compares that pattern with a reference to determine orientation with the reference. This method can be as simple as using the top of a visual field as a top reference. The information can be used to relate back to the position of a camera with respect to a body. In a desirable embodiment software accounts for the camera position with respect to an invasive device such as a hypodermic needle, a knife, a cutter, a liquid dispenser, a colloid dispenser, a fluid or air sucking device, or a needle/stitching device. In this embodiment, the software obtains a relationship between the positioning of the fiduciary device and the camera, has (or obtains) a relationship between the camera and the invasive device and then outputs a signal or command useful for controlling the invasive device.

Also disclosed herein is a camera for acquiring hyperspectral images comprising at least one lens and at least one sensor for detecting at least one characteristic of the mark. The camera will not acquire an image if misalignment, excessive changes in lighting due to internal or external sources, and/or contamination of subject is detected.

Other embodiments and advantages of the invention are set forth in part in the description, which follows, and in part, may be obvious from this description, or may be learned from the practice of the invention.

### Description of the Figure

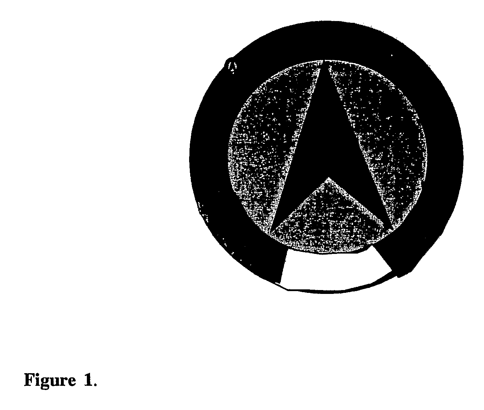

Figure 1 An illustrative example of an image reference according to the invention.

### Description of the Invention

When tissue being studied is stationary in time and space relative to the systems for illuminating and collecting radiation from the tissue, information collected relates to a single tissue sample - that common to the illumination and collection systems. When the tissue is permitted to move relative to the illumination and collection systems, the effect of such motion must be measured and included in the calculations. This invention concerns including in or on the tissue under study a mechanism for measuring the position of the tissue relative to the systems for illuminating it and collecting radiation from it and for incorporating material properties into this position-indicating mechanism that can be used to calibrate the wavelength selective parts of such system.

By way of example, Figure 1 shows a disposable mark according to an embodiment having a sharp black shape in the middle surrounded by an area of gray color. The outer band is subdivided into color bands that absorb at important wavelengths such as 550 nm (green), 570 nm (yellow), 760 nm (dark red) for oxy/deoxy and 970 nm (black) for water. Such mark as shown may include a bar code on its surface. The mark may be another shape such as an oval. The mark may comprise therapeutic material such as a pharmaceutical or scaffolding. The mark may have one or more transparent sections. The mark may have 1, 2, 3 or more light emitting diodes, and may have a power supply for the diodes. The mark may have one or more mirrored sections. Other variations readily will be appreciated.

In one embodiment of the invention, the detected and measured movement provides information regarding position of the fiducial mark, clinical information about the sample the mark is attached to such as the status of a nervous system, temperature information or other information as well. In an embodiment, two dimensional information from at least two different wavelengths is obtained for at least two different times and this information is compared with, for example stored data or stored algorithms to obtain a meaningful result. In an embodiment, the information is used to determine the accuracy of the fiducial mark. For example, a change in position from surrounding optical imaging of certain types, can indicate that a fiducial mark has fallen off. An algorithm implemented for this embodiment typically has one or more limit values such as maximum change in a border line, or maximum change in a surface. Upon detection of excessive movement with respect to one or more maximum limits, the software may trigger an alert, may simply remove data of the fiducial mark from further use, or change a parameter in an analysis. As another example, a color change of a fiducial mark as determined by spectral and/or dimensional change, can be used to determine temperature or humidity. This information can be used to compare with set or calculated limits, or used as one or more factors in a clinical evaluation. In another embodiment, such measured changes can be compared with stored measurements and determinations from earlier measurements to ascertain a change in clinical status.

Illuminating and viewing tissue with multiple wavelengths of light has been performed for many millions of years and has the process of sensing selected wavelengths of light. However, only in the past half century have orbiting satellites been able to collect images of the surface of the Earth at several wavelengths. Also recent are the algorithms that have been developed to use measurements from each voxel on the Earth's surface to classify the voxel into categories such as vegetation, animal tissue, water, metal, etc. An image collected at one or a narrow range of wavelengths contains properties of the voxels in its field of view at a small region of the possible wavelength range; multiple such images collected over distinct wavelength ranges can form a composite sometimes referred to as a multispectral or hyperspectral image. If the image of a tissue voxel is located in the same two dimensional location on each such image, then the images are said to be aligned and the multiplicity of images form a three dimensional cube referred to as a hypercube.

The measured properties of a single tissue voxel at multiple wavelengths are then the aggregate of data stored at the same spatial location on the multiple planes that compose the hypercube. In practice, the images at each wavelength range are collected at different points in time, and when the tissue moves between and/or during such image collections, the data at each spatial position in the hypercube will no longer be for a single voxel. However, in addition to variations in tissue position relative to the illuminating and collecting parts of the instrument, the intensity of the illumination may vary in time and space and with wavelength, and the transmission and sensitivity of the collection system may also vary with wavelength, time and position. There may also be additional confounding sources, such as environmental conditions like temperature and humidity, and shading and shadowing due to object shape as well as intrinsic variations within the tissue during the time that multiple images are collected.

Spatial patterns on objects have been used to register multiple images of the objects for many years. For some time, objects of known chemical composition have been placed in the common paths of illumination and viewing systems to measure their response with wavelength so as to compensate for variations thereof. For at least the past several decades, combinations of spatial registration and spectral calibration devices have been used in the color printing industry. A skilled artisan already has extensive and deep knowledge of the technology for developing and producing spatial and wavelength calibration devices and such details, while contemplated as embodiments of the invention, are too numerous for separate listing herein. As a representative sample of known techniques and devices intended for embodiments, the reader is directed to consider the disclosures of U.S. Nos. 6,940,999 ("Method for target detection and identification by using proximity pixel information"); 6,933,154 ("Optimal windows for obtaining optical data for characterization of tissue samples"); 6,750,964 ("Spectral imaging methods and systems"); Althof et al. (1997), "A rapid and automatic image registration algorithm with subpixel accuracy," IEEE Transactions on Medical Imaging, 16(3):308-316; Astrom et al. (1999), "Motion estimation in image sequences using the deformation of apparent contours," IEEE Transactions on Pattern Analysis and Machine Intelligence, 21(2):114-127; Dickman et al. (2001), "Identification of Cervical Neoplasia Using a Simulation of Human Vision," Journal of Lower Genital Tract Disease, 5(3):144-152; Eisner et al. (1987), "Use of Cross-Correlation Function to Detect Patient Motion During Spectral Imaging," Journal of Nuclear Medicine, 28(1):97-101; Ji et al. (2000), "Texture Analysis for Classification of Cervix Lesions," IEEE Transactions on Medical Imaging, 19(11):1144-1149; Schmid (1999), "Segmentation and Symmetry Measure for Image Analysis: Application to Digital Dermatoscopy," Ph.D. Thesis, Swiss Federal Institute of Technology (EPFL), Signal Processing Laboratory (LTS); Szeliski et al. (1997), "Spline-based image registration," International Journal of Computer Vision, 22(3):199-218; Thirion et al. (1999), "Deformation analysis to detect and quantify active lesions in three-dimensional medical image sequences," IEEE Transactions on Medial Imaging, 18(5):429-441; Treameau et al. (1997), "A Region Growing and Merging Algorithm to Color Segmentation," Pattern Recognition, 30(7):1191-1203; Weng et al. (1997), "Three-Dimensional Surface Reconstruction Using Optical Flow for Medical Imaging," IEEE Transactions on Medical Imaging, 16(5):630-641; Zheng et al. (1991), "Estimation of illumination direction, albedo, and shape from shading," IEEE Transactions on Pattern Analysis and Machine Intelligence, 13(7):680-702; and Zhengfang et al. (1998), "Identification of Colonic Dysplasia and Neoplasia by Diffuse Reflectance Spectroscopy and Pattern Recognition Techniques," Applied Spectroscopy, 52(6):833-8 39.

In considering the techniques and devices in the extant art, the technologies generally face six types of problems incurred while acquiring and interpreting hyperspectral information: 1) effective spatial resolution; 2) system spectral calibration; 3) external light variation; 4) focusing camera to achieve sharp images; 5) preventing infectious contamination of calibrator; and 6) effective examination of contoured surfaces. Other problems may include differences in surface temperature seen in thermal or infrared (IR) imaging (near infrared - NIR, short wave infrared - SWIR, far infrared - FIR, long wave infrared - LWIR), artifact detection and hydration. Yet other problem is the possible need to verify that polarization angle between emitted and received light has not changed.

A typical hyperspectral image acquisition takes some time - from milliseconds to minutes - depending on the amount of information to be obtained in each sampling process or "image" (using a liquid crystal tunable filter LCTF, acousto-optic tunable filter AOTF, diffraction grating, etc.). During this time, the observed surface (of a live subject/tissue) is very likely to move or alter. As a result, in many systems, more than one image plane is recorded sequentially. Consecutive images will observe slightly different areas and record slightly different images.

In other systems, (split optics, multiple cameras) light may be collected from different angles or go through different optical paths before being synthesized into a single image. The particular point on the tissue gets recorded at a pixel that has different coordinates on the camera consecutive frames. If spectra are plotted for each pixel, the spectral line will correspond to an area around that particular tissue point. Therefore, the final resolution of the spectral information is constrained not primarily by the resolution of the recording camera, but rather by involuntary motion by the live subject.

Movement of the tissue causes smearing of the hyperspectral image called "jitter." This problem is well known in imaging science. However, the main concern is changing of the actual "target properties" during the course of data collection. This results in creation of a virtual "error band" which is a function of the resolution of the measurement, the duration of the measurement, and the rate of change of the properties associated with the target (pixel or entire image).

Another type of error can arise due to polarization changes upon reflection. Accordingly, an embodiment monitors the quality of cross-polarization between emitted and received light. In an embodiment, polarization is used to detect a measurable quantity such as movement, temperature or positioning.

Embodiments alleviate problems with actual resolution via use of fiducial marks on the sample. United States Patent Nos. 6,810,279 and 6,081,612 teach such a method. Some researchers have used a marker to draw a dot or a cross. The stack of hyperspectral images is then realigned and cut, so that the dot or cross occupies the same pixel across the entire cube. Spatial resolution is constrained by the quality of registration across the images in a hypercube. Another way is to simply average the image, or calculate the movement and apply a "jitter" correction factor. An example of this method is the commercially available image stabilized camcorder. A possible problem with this is that the presented field of view is smaller than the actual field of view.

A second type of problem is system misalignment. Similar to preserving the spatial resolution, embodiments alleviate spectral misalignment of the system. Spectral calibration generally is necessary during acquisition for confirmation that at a given required wavelength the system is actually acquiring data at this wavelength, and it does not change during the acquisition. United States Patent Nos. 6,810,279, 6,081,612 and 5,924,981 discuss the importance of spectral calibration, however none of the calibration procedures are completed at the time of the actual data acquisition. They occur during calibration procedures, such as before or after the data are acquired. Moreover, many suggest doing it in two steps.

A first step includes a target that is nearly 100% opaque to electromagnetic radiation over a predetermined wavelength range. A second step includes spectral lamps or other illumination sources operating at discrete wavelengths. United States Patent Nos. 6,810,279 and 6,111,640 teach examples of such sources. Ideally, this calibration is performed just before and/or after each data collection step in the field to account for any changes in calibration of the spectrometer over time.

A third problem is that often, in the user environment (e.g., operating room, doctor's office, open field for military applications), it is impossible to completely control external light variations during data acquisition. Changes in external light intensity of spectral characteristic during acquisition may result in problems with interpretation of the hyperspectral data. It is especially cumbersome when light changes are not recorded. Some researches, such as United States Patent No. 6,810,279, suggest assessment of lighting conditions with a calibration pad. Others, such as United States Patent No. 6,081,612, argue for consistent measures of reflectance ratios in different spectral bands to account for variations in illumination pattern with wavelength, changes in the position of the illuminator, or aging of the lamp. However, the calibration occurs prior to the tissue measurement (see also United States Patent No. 5,924,981) and does not reflect the actual light variation during the data acquisition.

Fourthly, robust focusing of the system may be required to reach the best possible resolution. Manual focusing takes time and is not reliable. Once focused, the images might shift out of focus during acquisition due to subject and/or apparatus motion. Therefore it is necessary to control/adjust focus during acquisition. This can be facilitated by the presence of a well-defined (spectrally and spatially) fiducial mark in the field of view. "Spatial calibration" in all three dimensions including toward and away from the camera is highly important in evaluating sequential images in a series or images over time.

A fifth problem sometimes encountered is the need for a disposable calibration apparatus due to cleanliness requirements. This is two fold - single use devices assure sterility and prevents transmission of infection from patient to patient. Single use also assures spectral and spatial characteristics of the calibration device were not contaminated or distorted by previous use, especially in the hands of operators who are not highly skilled in performing technical procedures. This is critical to maintaining its function as a quality control device. When such an instrument is used with human or animal patients, it is essential to ensure that use of the instrument does not contaminate or infect a patient, and that there is no cross contamination between the patients. Similar concerns are addressed in United States Patent Nos. 6,810,279 and 5,924,981.

As shown in United States Patent No. 6,111,640, calibration to be performed prior to data acquisition and over the entire field of view of the instrument was covered by the calibrator so no signature of the observed tissue/subject is visible. Prior art teaches use of a calibration material that only withstands routine cleansing procedures. This cannot achieve the desired level of cleanliness or efficiency since it takes up time before each measurement and requires special resources.

Prior art discusses other types of calibration that are related to the ability of the apparatus to accurately record hemoglobin levels and relationships between oxy- and deoxy- hemoglobins. Such discussions were reflected in United States Patent No. 5,879,294 and included creating a library of various calibration curves established for different relative concentrations of a predetermined chromophore and/or different optical path length conditions, over a spectral region of interest.

A six type of problem that may be encountered is the concern over variations in contour. In addition to aforementioned aspects, assurance that lighting and camera work across the entire field of a complicated region of interest (ROI) is necessary. US Patent No 7,013,172 describes a calibration reference image pad that conforms to the shape of the image surface.

Spectroscopy is a powerful and useful tool for researchers in pursuit of many disease diagnoses. However, problems such as the ones mentioned herein present obstacles and frustrations in imaging systems use. Thus, there is an immediate need for safe, effective devices, systems and methods for acquiring and interpreting this valuable hyperspectral information.

As embodied and broadly described herein, the present invention is directed to hyperspectral imaging and calibration systems and devices and methods of use. Embodiments facilitate the following: non-invasive procedure, remote-sensing, access to previously unavailable information, earlier and more sensitive detection, detection of subclinical changes in tissue, definition of pathology, prediction of clinical outcomes, monitoring and adaptation of therapy, assessment of biomarkers related to hemoglobin and/or tissue hydration to provide useful metabolic information, and application to the assessment of local, regional or systemic diseases.

Hyperspectral imaging is a technique whereby a two-dimensional image is created that has spectral data inherent in each individual pixel. It is possible to correlate the spectrum of each pixel with the presence and concentration of various chemical species. This data can then be interpreted as a "gradient map" of species in a surface. This has been shown to have a high correlation with various physiologic and pathologic conditions when applied to various tissues.

The present invention alleviates one or more of the problems outlined above. An embodiment provides a single calibration device - a mark within the field of view - that is present during data acquisition. Certain characteristics are necessary for the desired level results. An embodiment provides a device containing a spatial pattern with a sharply peaked autocorrelation function in at least two directions, a set of reflectance standards at one or more wavelength bands, a means for attaching it to tissue so that its position on the tissue remains constant during data acquisition and a means for maintaining its clean or sterile properties, if any, until just before it is placed in the field of view.

In a desirable embodiment, the calibration mark is located, nearby, physically touching, or preferably located over, the surface of a calibration surface, such as, for example a calibrator described in U.S. No. 7,013,172 "Hyperspectral imaging calibration device." Desirably, the calibration mark area is a small portion (less than 50%, 25%, 10%, 5%, 2%, or even less than 1%) of the surface area of a calibration device and serves to improve precision and/or decrease error of imaging the calibration device. For example, a patient with skin or other tissue lesions may have one, two, three, four, five, six or more calibration devices covering clinically interesting portions of the patient body. The calibration devices may be attached over a sore, an open wound, or even become part of a scaffolding for repair. Fresh application of a calibration mark to an existing, worn calibration device allows a more accurate spectral measurement of the worn calibration device. In practice, a new calibration mark, with a unique identifier (bar code, set of sharp absorbance peaks, or any other kind of imaging feature) is removed from a package and may be affixed to the calibration surface. The mark provides an accurate and fresh reference for imaging and can help check for image degradation due to wear or accumulation of debris on the calibration surface. The mark may display individual information such as, for example, patient identification, body part or body tissue identification, time of day or calendar information, clinical treatment information, or doctor information. The imaging apparatus used to obtain spectral information may use the calibration mark as a quality check and merely discount or discard a measurement that indicates deterioration of the calibration surface.

Desirably, multiple calibration marks are packaged for use with a smaller number of calibration surfaces. For example, up to 12 marks may be packaged with one, two, three, four, five or six calibration surfaces. During use, a calibration surface may be affixed to a patient and a smaller calibration mark also added as a reference for imaging. After imaging, the smaller calibration mark may be removed. A later imaging of the calibration mark would be carried out after adding a second, fresh calibration mark. In this embodiment, the calibration marks desirably are disposable, used soon after removal from their package and provide a more reliable reference for imaging than features of the calibration surface itself, which are not renewed. Accordingly, one embodiment is a package of one calibration surface with multiple (for example, up to 5 or 12) calibration surfaces. Another embodiment provides a small number (for example, 2, 4, 6, 10, 12 or 15) of calibration surfaces with a larger number of calibration marks. In an embodiment, each calibration surface in a package (if multiple) has the same identification information, but each calibration mark is individually distinguishable. In this manner, the calibration mark provides identification information to a course of imaging treatment.

In one embodiment, the mark is small compared to a region of interest in the field of view of the hyperspectral imager ensuring that useful information is not obstructed. The mark is sterile and disposable after a single use to ensure the quality of reflection and avoid subject contamination. The preferred mark consists of a unique shape that has directional asymmetry. The shape is covered in a substance that absorbs strongly in all wavelengths considered. The shape is used to perform image registration and focusing, which can be done during data acquisition, before and/or after it. The mark also consists of an area with low reflectivity that has relatively flat absorption spectrum across all wavelengths considered. The signal recorded off such area is used to track variations in the external lighting during data acquisition as these variations impact on the data at the precise time of collection. The area may be less than 1cm, less than 8mm, between 6mm and 8mm, approximately 7mm, less than 3 mm, less than 2 mm, approximately 1 mm, or less than 1 mm, particularly in combination with a high resolution camera.

The mark can also consist of areas that are covered with a substance that reflects at the wavelengths particularly important in the data processing and the algorithm. These areas are used as all or part of the procedure for spectral calibration of the hyperspectral imager and can be present in varying concentrations. Depending on the number of wavelengths of interest, there is at least one or many areas used for spectral calibration. The calibration mark remains on the subject during data acquisition via adhesive properties of a side, preferably a back side, or by being ionized, or by being wet (using surface tension), or by any other mechanism of temporary or permanent attachment. In one embodiment, the calibration mark does not saturate with blood/fluid. In another embodiment the calibration mark tracks the presence and/or quantity of blood/fluid which accumulates over a random or specified period of time. It may or may not change with temperature or humidity, depending on design preferred. If one or more sensed signals such as the color or intensity of a mark on the calibration mark changes with at least one environmental or patient related factor, a change in the image is reported. Thus, an embodiment provides both a reference standard and a measurement. In an embodiment related to monitoring of a small area injury, the fiduciary device is both a reference and a therapeutic device such as for example, a bandage with fiduciary markings.

In a preferred embodiment, the shape or a printed object on the round or other smooth shape of the calibration device has sharp edges that can be used to determine the shape orientation and therefore, the subject orientation with respect to the camera. The sharpness of the shape is used to auto-focus the camera as well as to register images within the hypercube and across the entire measuring interval. Autofocus camera devices and software known to the skilled artisan are contemplated embodiments. Desirably, software is provided that accepts signal data, determines orientation (and optionally distance) from the camera, and determines an output signal from the comparison. The signal may be for example, represented on a video monitor, or as a signal to a computer or other electronic device.

In another embodiment, multiple hypercubes are acquired for long term observation purposes. The shape is colored with a distinct, pre-defined and unwavering color (e.g. black) that is highly absorbent in the entire viable spectral region, near infrared region, ultraviolet region or a combination of regions. An embodiment provides equal absorbency in visible and near infrared light, which facilitates registration and focusing. Preferably, there is a colored area with specific spectral characteristics around the shape. These spectral characteristics could be the spectrum of oxy- or deoxy- hemoglobin or water or any other chosen compound of interest. Preferably, different concentrations or ratios of dies either by mixing or by placing dies nearby and analyzing the two areas together to get an average concentration of the compound are used for a two or more point calibration. A few distinct colors in the area are used to calibrate the system with respect to chosen wavelengths. A gray area around the dark shape is used to record the intensity of the incoming light. The gray area has a flat spectrum, recorded in the systems' computer. Deviation from the predetermined spectra will be related to the changing external light conditions to provide feedback to data processing. This provides the possibility of quantitative readings of compounds of interest.

The colored mark may be of any non-toxic material including, but not limited to, ink, henna dye, marker, paint, vegetable dye, or any other such colorant. The mark can be generated from a chemical reaction, such as by adding an oxidant to an efficient luminescent material such as luminol or luciferin, and optionally with a catalyst such as a heme compound or luciferase. The chemical reaction may form a color as is known for a beta galactosidase, horseradish peroxidase, catalase alkaline phosphatase, or other enzyme with colorigenic substrate. In an embodiment, chemical generation of the optical mark is combined with chemical clinical treatment, and provides a reference or control of the chemical reaction itself, such as by using hydrogen peroxide to both treat and produce the fiduciary mark.

In a preferred embodiment, the device has an optical surface that is maintained in a stable form within a package before use and is removed from the package before application to a tissue. A software routine may be provided with the camera or other detecting equipment that monitors for decay in fidelity of the fiduciary mark. For example, a fluorescent reflection and reflection from one, two or more other colors may be compared by software to look for variable decay in signal, representing accumulation of dirt or grease that affects different colors differently. In an embodiment, the device is sterilized before use and sealed in packaging certified to maintain such sterility; in this embodiment the device may be sterilized and reused or may be disposable after a single use.

In a preferred embodiment, the device is manufactured in a "clean" environment before use and sealed in packaging certified to maintain such clean status; in this embodiment the device may be cleaned and reused or may be disposable after a single use. In the same or another embodiment, the spatial pattern of the shape or printed on the device is unique and has sharp edges that can be used to determine the shape orientation and therefore the subject orientation with respect to the camera. The high spatial-frequency content of the shape's spatial pattern may assist the operator to focus the camera as well as to register images within the hypercube and across the entire measuring interval (in case of a long term observation that requires acquisition of multiple hypercubes). The material of the portion of the device in contact with the patient is not toxic or allergenic to the tissue it is placed on, and may be opaque and highly absorbent over the wavelength range of 400 nanometers (nm) through 1000 nm 500 to 970, or 500 to 650 nm. The device may contain materials within or on it whose optical properties vary with wavelength. Again, materials may include, but are not limited to, ink, henna dye, marker, paint, vegetable dyes, or any other such colorant.

In one embodiment, such materials could have spectral absorbance properties similar to one or more of the pigments naturally present in the tissue. In this or another embodiment, some of the materials of the device could have optical properties that varied minimally with wavelength and could be used to estimate the strength of illumination and the orientation of the device in six dimensions (three spatial, three angular) with respect to the illuminating and viewing system.

To alleviate contour problems, at least one or more embodiments include using a flat surface or for screening purposes versus two or more at margins of an ulcer or complex wound or angled vantage point, or a ring with multiple fiduciaries around an ROI or a strip for the side with multiple fiduciaries. Another preferred method includes scanning with a laser bar code device as used in 3D stereo-lithography.

In one of any of the embodiments herein, the system may consist of a camera, a lens, an LCTF, a near real-time image display, a light source (LED array), and a sensor (movement, temperature).

In one of any of the embodiments herein, a camera is used for capturing images of the region of interest. The camera may be a single-chip or multiple-chip charge-coupled device which detects light in a plurality of spectral bands between the near ultraviolet to near infrared. The filter means may be a plurality of interference filters mounted on a wheel for stepping any filter into a position intercepting the light from the light source. Preferably, at least one of the spectral bands has a center which lies is less than 350 nanometers, between about 350 and 500 nanometers, at least one of the spectral bands has a center which lies between about 500 and 600 nanometers, at least one of the spectral bands has a center which lies between 600 and 700 nanometers, at least one other spectral band has a center which lies between about 750 and 1000 nanometers, and at least one other spectral band has a center which lies above 1000 nanometers.

A computer is used for receiving the digital images from the camera for processing. The computer may include a digital interface, a memory and a digital processor. A display is preferably provided as well. The computer may include an input to a digital interface for receiving the digital images. A memory stores the digital images, and the software controlling operation of the imaging system, the image processing, and the classification and characterization of the lesion. The digital processor, under control of the software, performs the calculations. The computer has an output connected to a display, which can display the processed images and the results of the classification and characterization procedures for each image. The computer also preferably has outputs connected to the source of light and the camera, for controlling their illumination level and exposure times.

Digital signals making up each of the digital images output from the camera are provided to the computer. The computer conducts image processing procedures on the digital images to calibrate the images, and to objectively segment, estimate parameters, and classify the lesions based on the estimated parameters. Operator judgment is not necessarily required at any point in the process.

Control is maintained by the computer over source intensity, filter position, and such camera settings as shutter timing, through the digital interface. Key control parameters are empirically chosen on the basis of feedback from histograms of trial images. The intensity of the lamp may be maintained at a stable value, commensurate with the dynamic range of the camera, by monitoring a secondary light source, connected electrically in series with the primary light source. The light output from the secondary source may be monitored by a light sensor that is optically isolated from light reflections associated with the primary source. Such reflections may be caused by the filters that are located on the filter wheel, or from the housing of the primary light source. This method provides optical feedback which is sensitive to changes in light intensity caused by changes in lamp lead resistance, for example, while it is insensitive to the variable amounts of light reflected from the filters, for example. By means of a closed control loop, the optical feedback from the secondary source may be used to maintain constant light output from the primary source. In addition, the lamp intensity may be further stabilized by monitoring light reflected from a material of stable reflectance. If the intensity of the light detected by the camera deviates from a predetermined desired value, the intensity of the output of the lamp can be adjusted. The power, voltage or current supplied to the lamp, may also be monitored.

Software and mathematical algorithms known to the skilled artisan are intended in embodiments for extracting the position of each tissue image at each time and wavelength range relative to the other images, and that compensate for variations in illumination and viewing conditions and for confounding influences. Such software and mathematical algorithms when applied to images of the device and living tissue provide the information necessary to calculate the tissue's structure, chemical composition and oxidative state. The hyperspectral imaging acquisition and calibration system as described in one or more of the embodiments including a camera, sensor, display, and computer applies algorithms to the hyperspectral camera to register and process the disposable calibration mark. This process allows for observation and application of the hyperspectral images acquired. Data can be plotted in three dimensional graphs and charts in addition to in-depth exploration of colored images.

Since the unaided eye cannot perceive light outside of the visible region or low-contrast structure in visible-light images, information which may be useful in diagnosing a lesion may not be directly observable. The present invention may be used in digital acquisition and processing of dermoscopic images. This improves diagnostic reliability by employing more of the information residing in such images that is not directly observable. For example, there have therefore been attempts to use objective, computer-based, image analysis algorithms that can discern meaningful differences between benign and malignant melanocytic lesions with sufficient accuracy.

Computer processing of images requires that the image be in digital form. A digital image is an array of digital signals whose values are a function of certain characteristics of the subject of the image. When imaging skin lesions, the digital images comprise digital signals whose values are a function of the re-emission characteristics of the skin and lesion, at different spectral bands of the light. The array is obtained by spatial sampling and quantizing the intensity of images obtained with film or directly by electronic cameras. Practical limitations on the number of picture elements or pixels per unit area of image determine the achievable spatial resolution of the digital image. The digital image typically needs to be segmented to separate the digital signals which are a function of the skin lesion from the digital signals which are a function of the surrounding skin.

Computer aided analysis has also been used to classify skin lesions using quantitative values indicative of particular characteristics of lesions, referred to as parameters. Based on histopathological diagnosis of lesions, algorithms have been developed which use linear or non-linear classifiers to combine parameters provided by the operator of an imaging device or a physician or computed by a processor, to yield a value which can be used to classify the lesion. Because some of the steps in the computer-aided analysis depend on subjective judgments of an individual, such analysis may provide highly variable results. Color photographic transparencies of skin lesions may be digitized and skin lesions may be directly imaged with "three-chip" digitizing cameras. Such cameras employ broad-band filter band passes that are ultimately based on the wavelength response of the human visual system and have large regions of overlap.

Electronics images may also be obtained in narrower, non-overlapping filter band passes, which may reveal additional, wavelength-dependent differences between the images of melanomas and of benign lesions. However, such devices have had poor resolution and/or poor signal-to-noise characteristics which prevent the acquisition of digital images of melanocytic skin lesions of sufficient quality for effective application of machine vision techniques for lesion diagnosis.

Existing imaging systems and processes also tend to suffer from an inability to provide the required repeatability of the value of extracted lesion parameters, due in part to a lack of standardization with respect to spatially varying artifacts, so that the parameters, therefore, lack invariance to lighting and image exposure conditions, for example. Obtaining high signal-to-noise ratios in images recorded in narrow filter band passes, when exposure times are sufficiently short that the skin is effectively "frozen" during the exposure sequence, has also been difficult. In addition, since the optimum wavelengths for automatic characterization may not be the optimum wavelengths for visual observation, it may be difficult to reconstruct high-fidelity color images from the digital images for visual interpretation by a clinician.

The present invention provides many benefits to both the patient and the medical provider. The patient will suffer less trauma and a faster recovery, fewer complications, and lower premiums. The medical provider gains speed in procedure and instrument preparation, lower fixed costs, and improved clinical outcomes. Additionally, quality of care is vastly improved.

The following examples illustrate embodiments of the invention, but should not be viewed as limiting the scope of the invention.

### Examples

Disposable calibration-fiducial mark is used during data acquisition on a diabetes subject, burn subject, or open surgery subject. Other areas of use include, but are not limited to, limited to, ischemia, claudication, assessment of safety of debridement, ***tissue grafts,*** shock, congestive heart failure, acute or chronic hemodynamic or vascular compromise, lymphoma, melanoma, basal cell carcinoma, therapeutic monitoring, radiation therapy, chemical-biological warfare, etc.

Hyperspectral data is acquired during open surgery on a breast cancer patient to perform an optical biopsy of the tumor bed after removal of the tumor. The subject is anesthetized and breathing. The mark is placed into cavity and hyperspectral data acquisition performed. In one embodiment, the imager uses the first few frames to locate the calibration mark using the predefined spatial and spectral properties to focus on its shape and to register consecutive images for each wavelength.

Once data are acquired, in one iteration, the hyperspectral imager uses spectral information recorded by pixels corresponding to the different areas of the calibration mark to spectrally calibrate the instrument and to account for external light variation, if any. The images are adjusted according to the calibration information and used for the final algorithm that outputs the pseudo-color images of the field of view onto a screen. This is all within a few seconds after the data acquisition is completed allowing a doctor to assess the tissue composition and make a diagnosis. Calibration image reference provides a quality control feature whereby all calibration criteria must be met before the image is delivered. In another iteration, the readings from the calibration target are used not to calibrate the instrument but to check to see if the instrument is in calibration. If it is not, preferably, the instrument will not take the reading or provide an error message.

In another example, hyperspectral image calibration occurs in two steps. Firstly, reference data are recorded that covers the entire field of view in a controlled fashion, typically just prior to imaging the tissue of interest. The reference data is used to process the images from the tissue of interest and include adjustments for the intensity and distribution of the illumination sources, collection efficiency of the optical system, and wavelength. Secondly, data are recorded from the image reference typically within a limited field of view, at the same time that data is recorded from the tissue of interest. In this way, reference data can be recorded in a controlled fashion that minimizes effects due to motion, ambient lighting, and imperfect focusing, and covers the entire field of view and wavelength space. The data from the image reference can then be used to ensure that the lighting is consistent with that measured in the first step, and check that any shifts in wavelengths, focusing and motion that occur during the time of measuring the tissue of interest is with an acceptable range and accounted for by the processing algorithm. If changes are outside an acceptable range, the user is prompted to retake the measurement.

A Medical HyperSpectral Imaging (MHSI) system was built and the technology applied in preclinical tests on a rat model of induced breast cancer. MHSI differentiated between tumors and small residual pieces of tumor from normal tissue. The human eye of an experienced animal surgeon has difficulty uniformly performing this function. Thus, the need for enhanced imaging such as the MHSI was needed. MHSI facilitates diagnosis of cancer tissue. As an imaging technology, MHSI carries an advantage of being useful as both a screening and a diagnostic tool. It can also be used for both lower and upper endoscopy. MHSI was also used for targeting the diagnosis of sessile polyps and Barrett's esophagus. Paired with an endoscopic tool (for example, one made by a medical device manufacturer such as one by Boston Scientific) the imaging system is capable of multiple applications assessing any body surface accessible by vision or tools to permit visualization of tissue..

On a shock patient, MHSI was performed using the following analysis techniques: average spectral index, heterogeneity index, spatial shape ("Mottling") index, and temporal shift index. The relationship between skin microcirculatory anatomy and regional blood flow heterogeneity was acquired and charted. An advantage of this new and powerful system is that MHSI can detect spatial features of subclinical shock induced mottling unlike single point measurements.

Other embodiments and uses of the invention will be apparent to those skilled in the art from consideration of the specification and practice of the invention disclosed herein. The term "comprising" as used throughout this application includes the more limiting terms and phrases "consisting essentially of" and "consisting." It is intended that the specification and examples be considered exemplary only.

## Claims

1. A disposable imaging reference for hyperspectral imaging of a tissue surface comprising a tissue compatible object having at least a first surface for contacting the tissue and at least a second surface having a characteristic pattern of sharp contrast color peaks, wherein a first sharp contrast color peak in the characteristic pattern is in the middle of the second surface and is a unique shape having directional asymmetry,
the first sharp contrast color peak is surrounded by an area of low reflectivity that has a flat absorption spectrum, and **characterized in that** an outer band surrounds the area of low reflectivity, the outer band being subdivided into bands that have two or more of the sharp contrast color peaks in the characteristic pattern and that have different identifying constant color standards at one or more wavelength bands.

2. The imaging reference of claim 1, further comprising a means for affixing the reference to the tissue and later removing the reference for disposal.

3. The imaging reference of claim 2, wherein the means for affixing the reference and removing the reference is an adhesive on said first surface.

4. The imaging reference of claim 1, wherein the sharp contrast color comprises sharp contrast reflectances.

5. The imaging reference of claim 1, wherein one or more of the color standards are light emitters.

6. The imaging reference of claim 5, wherein the light emitters are selected from the group consisting of one or more chemiluminescence compounds, one or more phosphorescent compounds, one or more radioactivity compounds and one or more scintillants, one or more fluorescent compounds, one or more electroluminescent emitters, and one or more light emitting diodes.

7. The imaging reference of claim 1, wherein
the second surface sharp contrast color peaks provide an unvarying wavelength and color intensity signal for at least one minute when exposed to the atmosphere; or
the second surface sharp contrast color peaks provide an unvarying wavelength and color intensity signal for at least 10 minutes when exposed to the atmosphere.

8. The imaging reference of claim 1, wherein the first surface for contacting the tissue has a longest dimension of less than 1 cm.

9. The imaging reference of claim 1, further comprising a calibration pad.

10. The imaging reference of claim 9, wherein the calibration pad is separated from the imaging reference by at least 1 cm.

11. A labile disposable imaging reference system comprising the imaging reference of any preceding claim, and one or more chemical agents that, when in contact with the second surface, destroys one or more color properties of the second surface.

12. A method for aligning hyperspectral images comprising aligning images with the imaging reference of claim 1 and acquiring images from between one and at least four wavelengths.

13. The method of claim 12, further comprising:
the step of adjusting the images with an algorithm; and/or
the step of adjusting the images with a measurement from a separate calibration pad taken in addition to (prior, after, simultaneous with) collection of measurement from patient; and/or
the step of comparing spectra obtained from the calibration pad and from the image reference to determine a change in lighting.

14. The imaging reference of claim 1, wherein the second surface further comprises an area of reflectivity that changes in response to changes in surface temperature.

15. The imaging reference of claim 1, wherein
a first of the two or more sharp contrast color peaks in the characteristic pattern absorbs at 550 nm, 570 nm or 760 nm,
a second of the sharp contrast color peak in the characteristic pattern of sharp contrast color peaks absorbs at 970 nm.

16. The imaging reference of claim 1, wherein the first sharp contrast color peak is black and the area of low reflectivity is grey.

## Patentansprüche

1. Eine Einweg-Bildgebungsreferenz für hyperspektrale Bildgebung einer Gewebeoberfläche, umfassend ein gewebekompatibles Objekt mit mindestens einer ersten Oberfläche zum Kontaktieren des Gewebes und mindestens einer weiteren Oberfläche mit einem charakteristischen Muster von Farb-Peaks mit starkem Kontrast, wobei ein erster Farb-Peak mit starkem Kontrast in dem charakteristischen Muster sich in der Mitte der zweiten Oberfläche befindet und eine eindeutige Form mit gerichteter Asymmetrie ist,
der erste Farb-Peak mit starkem Kontrast von einem Bereich mit niedriger Reflektivität umgeben ist, der ein flaches Absorptionsspektrum hat, und
dadurch charakterisiert, dass ein äußeres Band den Bereich mit niedriger Reflektivität umgibt, wobei das äußere Band in Bänder unterteilt ist, die zwei oder mehr Farb-Peaks mit scharfem Kontrast in dem charakteristischen Muster haben und die verschiedene Identifizierungskonstanten-Farbstandards bei einer oder mehreren Wellenlängen-Banden haben.

2. Die Bildgebungsreferenz von Anspruch 1, außerdem umfassend ein Mittel zur Befestigung der Referenz an dem Gewebe und späteren Entfernung der Referenz zur Entsorgung.

3. Die Bildgebungsreferenz von Anspruch 2, wobei das Mittel zur Befestigung der Referenz und Entfernung der Referenz ein Klebstoff auf jener ersten Oberfläche ist.

4. Die Bildgebungsreferenz von Anspruch 1, wobei die Farbe mit scharfem Kontrast Reflektivitäten mit scharfem Kontrast umfasst.

5. Die Bildgebungsreferenz von Anspruch 1, wobei einer oder mehrere der Farbstandards Licht-Emitter sind.

6. Die Bildgebungsreferenz von Anspruch 5, wobei die Licht-Emitter ausgewählt sind aus der Gruppe bestehend aus einer oder mehreren chemolumineszenten Verbindungen, einer oder mehreren phosphoreszierenden Verbindungen, einer oder mehreren radioaktiven Verbindungen und einem oder mehreren Szintillatoren, einer oder mehreren fluoreszierenden Verbindungen, einem oder mehreren elektroluminszenten Emittern und einer oder mehreren licht-emittierenden Dioden.

7. Die Bildgebungsreferenz von Anspruch 1, wobei
die Farb-Peaks mit starkem Kontrast der zweiten Oberfläche ein gleichbleibendes Wellenlängen- und Farbintensitätssignal für mindestens eine Minute liefern, wenn sie der Atmosphäre ausgesetzt werden; oder
die Farb-Peaks mit starkem Kontrast der zweiten Oberfläche ein gleichbleibendes Wellenlängen- und Farbintensitätssignal für mindestens zehn Minuten liefern, wenn sie der Atmosphäre ausgesetzt werden.

8. Die Bildgebungsreferenz von Anspruch 1, wobei die erste Oberfläche zum Kontaktieren des Gewebes eine längste Abmessung von weniger als 1 cm hat.

9. Die Bildgebungsreferenz von Anspruch 1, außerdem umfassend ein Kalibrierungsfeld.

10. Die Bildgebungsreferenz von Anspruch 9, wobei das Kalibrierungsfeld vom der Bildgebungsreferenz mindestens 1 cm getrennt ist.

11. Ein instabiles Einweg-Bildgebungsreferenz-System, umfassend die Bildgebungsreferenz eines der vorhergehenden Ansprüche und ein oder mehrere chemische Agentien, das, wenn es in Kontakt mit der zweiten Oberfläche ist, eine oder mehrere Farbeigenschaften der zweiten Oberfläche zerstört.

12. Ein Verfahren zur Anpassung hyperspektraler Bilder, umfassend die Anpassung von Bildern mit der Bildgebungsreferenz von Anspruch 1 und Aufnahme von Bildern von zwischen einer und mindestens vier Wellenlängen.

13. Das Verfahren von Anspruch 12, außerdem umfassend:
den Schritt der Justierung der Bilder mit einem Algorithmus; und/oder den Schritt der Justierung der Bilder mit einer Messung von einem separaten Kalibrierungsfeld, aufgenommen zusätzlich zu(vorher, nachher, gleichzeitig mit) der Erfassung der Messung von einem Patient; und/oder
den Schritt des Vergleichs von Spektren, die von dem Kalibrierungsfeld und von der Bildgebungsreferenz erhalten wurden, um eine Änderung der Beleuchtung zu bestimmen.

14. Die Bildgebungsreferenz von Anspruch 1, wobei die zweite Oberfläche außerdem einen Bereich mit Reflektivität umfasst, die sich als Reaktion auf Veränderungen der Umgebungstemperatur ändert.

15. Die Bildgebungsreferenz von Anspruch 1, wobei
ein erster der zwei oder mehr Farb-Peaks mit scharfem Kontrast in dem charakteristischen Muster bei 550 nm, 570 nm oder 760 nm absorbiert,
ein zweiter des Farb-Kontrast-Peaks mit scharfem Kontrast in dem charakteristischen Muster von Farb-Peaks mit scharfem Kontrast bei 970 nm absorbiert.

16. Die Bildgebungsreferenz von Anspruch 1, wobei der erste Farb-Peak mit scharfem Kontrast schwarz ist und der Bereich von niedriger Reflektivität grau ist.

## Revendications

1. Repère d'imagerie jetable pour l'imagerie hyperspectrale d'une surface de tissu comprenant un objet compatible avec un tissu ayant au moins une première surface pour entrer en contact avec le tissu et au moins une deuxième surface ayant un schéma caractéristique de pics de couleur fortement contrastés, dans lequel
un premier pic de couleur fortement contrasté dans le schéma caractéristique est au milieu de la deuxième surface et est une forme unique ayant une asymétrie directionnelle,
le premier pic de couleur fortement contrasté est entouré d'une zone de faible réflectivité qui a un spectre d'absorption uniforme, et
**caractérisé en ce qu'**une bande extérieure entoure la zone de faible réflectivité, la bande extérieure étant subdivisée en bandes qui ont deux ou plusieurs pics de couleur fortement contrastés dans le schéma caractéristique et qui ont différents standards d'identification de couleur constante sur une ou plusieurs bandes de longueur d'onde.

2. Repère d'imagerie selon la revendication 1, comprenant en outre un moyen de fixation du repère au tissu et ultérieurement, de retrait du repère pour l'éliminer.

3. Repère d'imagerie selon la revendication 2, dans lequel le moyen de fixation du repère et de retrait du repère est un adhésif sur ladite première surface.

4. Repère d'imagerie selon la revendication 1, dans lequel la couleur fortement contrastée comprend des réflectivités fortement contrastées.

5. Repère d'imagerie selon la revendication 1, dans lequel un ou plusieurs des standards de couleur sont des émetteurs de lumière.

6. Repère d'imagerie selon la revendication 5, dans lequel les émetteurs de lumière sont choisis dans le groupe constitué d'un ou plusieurs composés chimioluminescents, un ou plusieurs composés phosphorescents, un ou plusieurs composés radioactifs et ou un plusieurs agents scintillants, un ou plusieurs composés fluorescents, un ou plusieurs émetteurs électroluminescents et une ou plusieurs diodes lumineuses.

7. Repère d'imagerie selon la revendication 1, dans lequel
les deuxièmes pics de couleur de surface fortement contrastés fournissent une longueur d'onde et un signal d'intensité de couleur invariables pendant au moins une minute lorsqu'ils sont exposés à l'atmosphère ; ou
les deuxièmes pics de couleur de surface fortement contrastés fournissent une longueur d'onde et un signal d'intensité de couleur invariables pendant au moins 10 minutes lorsqu'ils sont exposés à l'atmosphère.

8. Repère d'imagerie selon la revendication 1, dans lequel la première surface pour entrer en contact avec le tissu a une dimension la plus longue de moins de 1 cm.

9. Repère d'imagerie selon la revendication 1, comprenant en outre un bloc d'étalonnage.

10. Repère d'imagerie selon la revendication 9, dans lequel le bloc d'étalonnage est séparé du repère d'imagerie d'au moins 1 cm.

11. Système de repère d'imagerie jetable labile comprenant le repère d'imagerie selon l'une quelconque des revendications précédentes, et un ou plusieurs agents chimiques qui, lorsqu'ils sont en contact avec la deuxième surface, détruisent une ou plusieurs propriétés de coloration de la deuxième surface.

12. Procédé d'alignement d'images hyperspectrales comprenant l'alignement d'images avec le repère d'imagerie selon la revendication 1 et l'acquisition d'images à partir d'entre une et au moins quatre longueurs d'onde.

13. Procédé selon la revendication 12, comprenant en outre :
l'étape d'ajustement des images avec un algorithme ; et/ou
l'étape d'ajustement des images avec une mesure d'un bloc d'étalonnage séparé pris en plus (avant, après, simultanément) du recueil d'une mesure du patient ; et/ou
l'étape de comparaison de spectres obtenus à partir du bloc d'étalonnage et à partir du repère d'imagerie pour déterminer un changement de luminosité.

14. Repère d'imagerie selon la revendication 1, dans lequel la deuxième surface comprend en outre une zone de réflectivité qui change en réponse à des changements de la température de surface.

15. Repère d'imagerie selon la revendication 1, dans lequel
un premier des deux ou plusieurs pics de couleur fortement contrastés dans le schéma caractéristique absorbe à 550 nm, 570 nm ou 760 nm,
un deuxième du pic de couleurs fortement contrasté dans le schéma caractéristique absorbe à 970 nm.

16. Repère d'imagerie selon la revendication 1, dans lequel le premier pic de couleur fortement contrasté est noir et la zone de faible réflectivité est grise.
